(19) Europäisches Patentamt
European Patent Office
Office européen des brevets

(11) **EP 1 991 125 B1**

(12) **FASCICULE DE BREVET EUROPEEN**

(45) Date de publication et mention
de la délivrance du brevet:
**21.10.2009 Bulletin 2009/43**

(21) Numéro de dépôt: **07731072.0**

(22) Date de dépôt: **02.03.2007**

(51) Int Cl.:
***A61B 5/107*** *(2006.01)*

(86) Numéro de dépôt international:
**PCT/FR2007/000372**

(87) Numéro de publication internationale:
**WO 2007/099235 (07.09.2007 Gazette 2007/36)**

(54) **PROCÉDÉ ET DISPOSITIF DE DÉTERMINATION DES PLAGES DE PRESSION DEVANT ÊTRE EXERCÉES PAR UNE ENVELOPPE DE CONTENTION OU COMPRESSION**

VERFAHREN UND VORRICHTUNG ZUR BESTIMMUNG DER DRUCKSPANNEN, DIE VON EINER SCHIENE ODER EINEM KOMPRESSIONSVERBAND AUSGEÜBT WERDEN MÜSSEN

METHOD AND DEVICE FOR DETERMINING THE RANGES OF PRESSURE TO BE EXERTED BY A SPLINTING OR COMPRESSION BANDAGE

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **02.03.2006 FR 0601864**

(43) Date de publication de la demande:
**19.11.2008 Bulletin 2008/47**

(73) Titulaire: **Couzan, Serge**
**42000 Saint-Etienne (FR)**

(72) Inventeur: **Couzan, Serge**
**42000 Saint-Etienne (FR)**

(74) Mandataire: **Perrier, Jean-Pierre**
**55 rue Barthélemy Villemagne**
**42340 Veauche (FR)**

(56) Documents cités:
**WO-A2-98/55072          DE-A1- 3 223 711**
**FR-A- 2 682 279          FR-A1- 2 795 941**
**US-A- 3 552 382          US-A- 4 502 301**
**US-A- 4 605 010**

• **MARSHALL: "Compression therapy in leg ulcer management." NURSING STANDARD, vol. 15, no. 22, février 2001 (2001-02), pages 64-70, XP002408433**

EP 1 991 125 B1

**Description**

L'invention est relative à un procédé et à un dispositif de détermination des plages de pression devant être exercées par une enveloppe de serrage, pour contention ou compression, pour parvenir aux mêmes états d'un élément du corps d'un être vivant que ceux observés et définis pendant une mesure avec un équipement d'imagerie médicale.

La contention élastique est bien connue depuis les années 1950 pour le traitement des pathologies de la circulation veineuse et plus spécialement des insuffisances veineuses. Dans ces applications, une enveloppe en tricot élastique, obtenue par tricotage circulaire ou par tricotage plan et assemblage des bords du lé, est disposée autour du membre inférieur ou supérieur à traiter pour exercer sur celle-ci une contention-compression. L'augmentation de la pression tissulaire qui en résulte à pour but de réduire le calibre des veines, mais aussi le reflux, tout en accélérant la vitesse circulatoire. Ce but n'est pas toujours atteint avec les enveloppes actuelles.

Divers autres traitements du corps humain mettent en oeuvre la contention élastique. Il en est ainsi pour le traitement :

- de la cellulite, par compression de la masse adipeuse ;
- des muscles, de l'aponévrose et des tendons, après claquage, tendinite ou oedème, entraînant inflammations et douleurs,
- dans le système artériel et veineux, par exemple pour réduire fatigues, lourdeurs, crampes et douleurs en favorisant l'élimination des toxines, ou pour limiter et réduire la stase veineuse, l'hyperpression, l'oedème et ainsi le risque de formation d'une thrombose.
- dans le système lymphatique pour évacuer le liquide de stase responsable des lymphoedèmes.

En théorie, la pression P exercée sur le membre par l'enveloppe élastique est déterminée par la loi de Laplace dont l'expression est :

$$P = T/R,$$

dans laquelle T est égale à la valeur de la tension de l'enveloppe,
et R est égal au rayon de courbure de la surface comprimée, donc du membre traité.

En pratique, la tension de l'enveloppe dépend :

- de la tension donnée au fil de guipage entourant les fils ou brins élastiques, en gomme naturelle ou en matière synthétique,
- de la grosseur et du serrage de la maille du tricot,
- et, surtout, puisque ces deux premiers paramètres sont en général constant dans un processus de fabrication en série, de la tension donnée au fil ou brin élastique guipé à l'entrée de la machine à tricoter, cette valeur étant la seule pouvant être modifiée pendant le fonctionnement de la machine à tricoter.

Pour faciliter la prescription par le corps médical et simplifier la fabrication des bas à varices, les diverses contentions pouvant être prescrites ont été rangées dans plusieurs plages de valeurs, déterminées par mesure des pressions de contention obtenues sur un modèle de jambe ayant une cheville de section circulaire et une conformation « standard ». Ces plages sont dénommées Classes et s'étendent entre des valeurs extrêmes exprimées en millimètres de mercure (mm de Hg) ou en Pascal,

Avec ce système de classement, on considère qu'un bas porté par le patient satisfait à la prescription s'il exerce sur la partie de corps une contention dont la valeur est comprise dans la plage définie entre les deux valeurs extrêmes de la classe prescrite. Avec des classes ayant des plages de valeurs très étendues, puisque allant du quart à la moitié de l'une des valeurs extrêmes, le recours à ces classes amène une approximation importante de la valeur de contention théorique qu'il conviendrait d'exercer sur la partie du corps pour obtenir l'effet médical espéré.

Il résulte de cette façon de procéder que si le port de l'enveloppe élastique procure une contention, sa valeur réelle reste incertaine et ses effets peuvent être excessifs, insuffisants et voir même néfastes, par exemple en créant des douleurs par serrage d'un nerf, ou par effet garrot en réduisant le calibre d'une artère. Cela est d'autant plus regrettable que le seul moyen de vérifier ces effets avec la contention en place sur la partie du corps traitée est l'examen par imagerie par résonance magnétique nucléaire ou par tomodensimétrie (Scanner), examens qui sont coûteux et ne peuvent être mis en oeuvre que par des moyens volumineux, non transportables.

Cette incertitude se retrouve dans tous les traitements actuels par contention, élastique ou non, traitements dont l'efficacité dépend :

- d'une part, de la profondeur de l'élément devant être contraint dans la partie du membre ou du corps,
- et d'autre part, de l'épaisseur, de la nature et de la résistance de la masse absorbante formant interface et s'étendant entre la peau et l'élément du corps devant être traité.

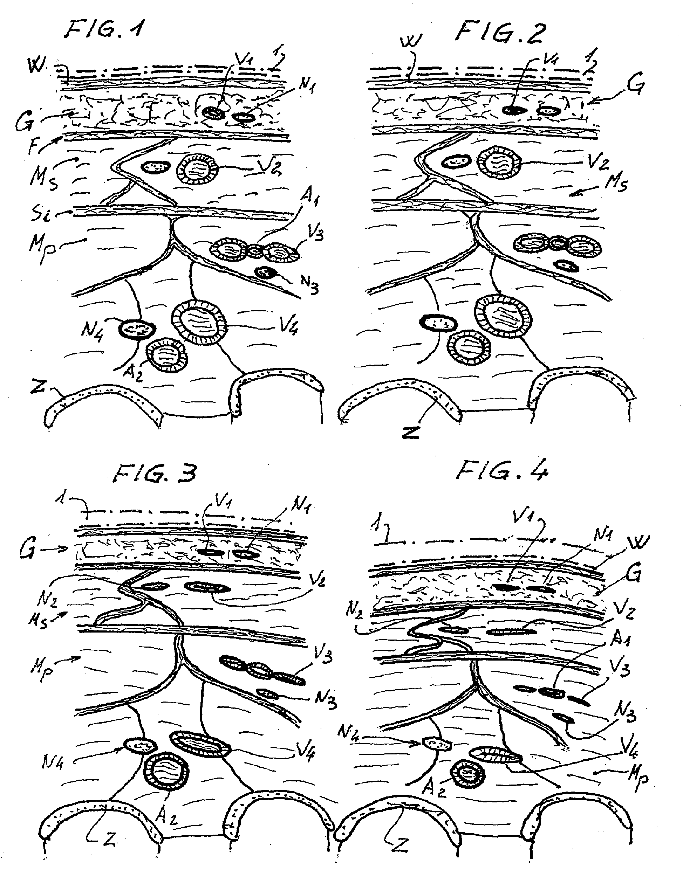

L'influence de ces paramètres est montrée sur les figures 1 à 4 annexées qui représentent des copies d'écran lors de l'examen de la section transversale d'une jambe, au niveau du creux poplité, par un échographe dont la sonde est passée sur la fenêtre acoustique d'un brassard gonflable du type décrit dans le document WO 01/03584. Ces figures illustrent l'évolution de la compression en allant, respectivement, d'une valeur de serrage nulle à une valeur comprise entre 50 et 80 mm de mercure.

Dans ce dessin, les veines portent la référence V, les artères celle A et les nerfs celle N. La peau W recouvre le tissu graisseux sous cutané G. Le fascia F sépare ce tissu du muscle superficiel Ms, lui même séparé du muscle profond Mp par le septum intermusculaire Si. Enfin, C désigne du cartilage et Z l'os des condyles de l'articulation du genou.

A la figure 1, le brassard gonflable 1 d'un sphygmanomètre est gonflé sous une faible pression. Il n'exerce aucun effet de serrage sur le membre, sur le tissu sous cutané G et sur la veine V1.

A la figure 2, la valeur de compression exercée sur le membre par le brassard 1 est de l'ordre de 15 mm de mercure. Seule la veine V1 disposée dans le tissu graisseux sous cutané G est comprimée, les autres éléments ne subissent pas de variation visible. La même mesure effectuée sur une autre personne avec la même valeur de pression dans le brassard peut avoir pour effet soit une légère compression de la veine V2, si le tissu G à une très faible épaisseur, soit aucune compression si le tissu G est très épais et tend à répartir l'effort de compression et de pénétration sur tout la surface de la zone de mesure.

A la figure 3, correspondant à une compression comprise entre 20 et 50 mm de mercure, les veines V2 et V3; respectivement dans le muscle superficiel Ms et dans le muscle profond Mp, sont comprimées, de même que le nerf N1 du tissu sous cutané G et, à un moindre degré, les nerfs N2 du muscle superficiel Ms. La veine V4 dans le muscle profond est rétreinte, sans être fermée.

Au-delà de 50 à 60 mm de mercure de compression et comme montré à la figure 4, les veines V1 à V3 et l'artère A1 sont très comprimées et se collabent. Les nerfs N1, N2 et N3, sont tous fortement comprimés, et plus faiblement pour le nerf profond N4. La veine profonde V4 est également comprimée. Elle se collabe vers 80 à 90 mm de mercure.

L'artère profonde A2 est relativement préservée et commence à être comprimée vers 100 - 110 mm de mercure, mais cela varie en fonction de la tension artérielle de l'individu.

On observe que pour toute personne de taille et de poids moyen, et en l'absence de maladie, les valeurs données pour les veines sont relativement constantes.

Il ressort de ce qui précède que la transmission de l'effort de pénétration dans la masse absorbante dépend de nombreux facteurs personnels, tels que profondeur des vaisseaux dans le membre, morphologie et nature de la masse absorbante formé par le tissu graisseux sous cutané G, les muscles superficiels Ms et les muscles profonds Mp, de sorte qu'il est illusoire de réaliser un traitement efficace par contention élastique à partir d'enveloppes fabriquées en série avec des tolérances de fabrication larges, car la contention réellement transmise au corps est, en générale, très approximative par rapport à la valeur nécessitée pour le traitements.

Cette approximation de la valeur réelle de la contention exercée sur le corps limite l'application de ce type de traitement, alors même qu'il pourrait être utilisé avec des enveloppes peu ou non élastiques, fournissant des contentions plus constantes dans le temps car moins sensibles à l'affaiblissement des fils de la structure tissée.

L'objet de l'invention est de fournir un procédé et un dispositif permettant de quantifier, pour chaque individu et quelle que soit sa morphologie, la valeur de la compression devant être exercée sur un membre ou une autre partie du corps, pour obtenir avec une enveloppe de contention ou de compression, un changement d'état de l'un des éléments inclus dans la masse absorbante, tel que compression d'un muscle, pour réduire une douleur ou fatigue, et cela avec la certitude de ne pas générer de douleurs ou d'autres effets indésirables, par exemple par serrage d'un nerf ou d'une artère.

Le procédé est mis en oeuvre avec un équipement d'imagerie médicale comprenant des moyens d'émission de signaux, d'ondes, ou de rayonnements à travers un brassard gonflable entourant la partie de corps comportant l'élément à analyser, des moyens de traitement des signaux, ondes ou rayonnements captés ou réfléchis et un écran de visualisation en image des signaux traités.

Comme dans les documents WO 0103584 et WO98/55072, le procédé comprend les phases suivantes :

- a) mise en place du brassard) sur la partie du corps à examiner et émission des signaux, ondes ou rayonnements à travers lui,
- b) repérage sur l'écran de l'élément à analyser,
- c) gonflage du brassard jusqu'à ce que le repérage à l'écran de l'élément en cours d'examen fasse apparaître une modification de l'état de cet élément,

- et d) lecture et mémorisation de la pression relevée dans le brassard pour parvenir à cet état,

Le procédé selon l'invention se distingue de cet état de la technique en ce que ses phases sont aménagées de la façon suivante :

- pendant la phase b), repérage sur l'écran des éléments structurels correspondant à la masse absorbante formant interface entre la peau et l'élément à analyser et pouvant comprendre les tissus sous cutanés, les tissus graisseux, les aponévroses, les fascias, les muscles, les tendons, les structures nerveuses, vasculaires et/ou ostéo-cartilagineuses,
- pendant la phase c) arrêt du gonflage dès le début de modification de l'état de l'élément analysé, puis lecture et mémorisation de la valeur de la pression PO qui traverse la masse absorbante d'interface,
- après mémorisation de la valeur P0, reprise du gonflage, jusqu'à la visualisation à l'écran de la modification souhaitée de l'état de l'élément analysé, puis arrêt de ce gonflage et lecture et mémorisation de la valeur de la pression P1 relevée dans le brassard pour parvenir à cet état,
  tandis que sont ajoutées les phases suivantes
- e) mesure et mémorisation de la longueur circonférentielle L de la partie examinée du corps,
- et f) transfert des valeurs mémorisées de P0, P1 et L, soit vers un support magnétique, soit vers les moyens de fabrication d'une enveloppe élastique de contention, soit vers les moyens de mise en tension d'une enveloppe non élastique.

Il faut remarquer ici que, à la différence du processus de mesure de la tension sanguine avec un tensiomètre, procédé selon lequel la première mesure s'effectue à la réouverture du vaisseau préalablement fermé par la coussin gonflable, le procédé selon l'invention assure la mesure de la valeur de la pression P0 à la première modification d'état de l'élément analysé, pour déterminer une valeur de seuil, et ne relève pas la valeur d'une pression sanguine. Il en est de même pour la valeur de pression P1, qui est relevée quand l'élément observé atteint l'état recherché, par exemple compression d'un muscle sans génération de douleurs par compression de nerfs ou d'artère, donc sans effet délétère pour le patient.
Ce procédé se distingue aussi du document WO98/55072 décrivant un procédé et un dispositif dans lesquels un brassard exerce sur un membre une compression externe ayant pour fonction de réduire le diamètre d'une veine et le reflux veineux en prévision d'un traitement médical par sonde thermique visant à restaurer les fonctions des valvules proches de la zone traitée. Dans cette application, la pression est nécessaire mais ne fait pas l'objet d'un relevé pouvant servir ultérieurement, d'autant plus que le traitement est nécessairement simultané.
La connaissance précise de la pression P1 nécessaire à l'obtention du changement d'état ou de l'effet attendu garantit l'efficacité du traitement ultérieur avec une enveloppe élastique fournissant cette compression, sans risque de la dépasser avec des effets traumatisants, tandis que la connaissance de la pression de seuil P0 permet d'éliminer toute contention insuffisante.
Il en est ainsi pour le traitement de la cellulite dans lequel l'écrasement des graisses est réalisé, grâce à l'invention, sans incidences néfastes sur les réseaux vasculaires superficiels et profonds et sur les structures nerveuses. De même, la contention fournie par une enveloppe, fabriquée à partir des données relevées par le procédé selon l'invention, pour serrer un tendon en vue de réduire l'inflammation et la douleur, est parfaitement adaptée au besoin, sans risque d'être inutile ou excessive.
Dans une forme de mise en oeuvre, visant à déterminer la tension qu'il faut donner aux fils élastiques d'une enveloppe élastique devant procurer un effet de contention ou de compression de valeur déterminée, le procédé comprend, une phase supplémentaire g), réalisée pendant la mesure ou lors de la fabrication de l'enveloppe, et consistant à appliquer à l'équation E :

$$T = P \times L / 2\pi,$$

les valeurs mémorisées de P0, P1 et de L pour déterminer les valeurs de seuil T0 et optimale T1 de la tension T qu'il faut donner aux fils élastiques de l'enveloppe pour obtenir la contention ou compression produisant les mêmes effets que ceux repérés à l'écran pendant la mesure.
Ainsi, la mesure garantit non seulement l'obtention de l'effet attendu, mais, en fournissant immédiatement la valeur de seuil T0 et la valeur optimale T1 de la tension qu'il faut donner aux fils, simplifie la fabrication et s'oppose à toutes dérives, autres que celles dues aux nécessaires tolérances de fabrication.
Pour son application à la régulation de la circulation vasculaire, veineuse ou artérielle, le procédé de mesure consiste, pour un même tronçon de membre ou de corps :

- à mesurer d'abord la valeur P0 mini à partir de laquelle le vaisseau examiné est soumis à un début de réduction de sa section,
- à déterminer par l'équation E la valeur de la tension minimale T0 à donner ultérieurement aux fils de l'enveloppe,
- à mémoriser la valeur T0,
- à reprendre le gonflage du coussin du brassard jusqu'à ce que la dimension transversale du vaisseau examiné, observé sur l'écran de l'équipement d'imagerie, atteigne un taux de réduction, compris entre 10 et 100 %, choisit en fonction du traitement,
- à lire et mémoriser la valeur de la pression P1 mesurée dans le brassard,
- et à déterminer par le calcul avec l'expression E, et avec la même valeur de L mais avec la valeur P1 de la pression, la valeur de la tension T1 qu'il faudra communiquer aux fils élastiques pour obtenir, par l'enveloppe compressive, la réduction de calibre du vaisseau perçue à l'écran.

Ce procédé permet de déterminer les tensions optimales devant être communiquée aux fils élastiques de l'enveloppe compressive dans une zone précise du membre examiné, et cela quelle que soit la position, superficielle ou profonde, du vaisseau examiné et quelle que soit l'importance de la masse absorbante gênant la compression du vaisseau. La mesure est effectuée par une procédure simple, rapide et non invasive, puisque voisine de celle utilisée pour prendre connaissance de la pression sanguine du patient.

De préférence, les phases respectivement :

- b) à d) de mesure de la valeur de la pression P dans le coussin gonflable, pour un ou plusieurs états de l'élément examiné,
- la phase e) de mesure circonférentielle L du membre
- et la phase g) de détermination des tensions T0 et T1, définissant la plage de contention ou compression,

sont réalisées successivement, d'une part, sur chacune des extrémités de la partie examinée du membre ou du corps et, d'autre part, au moins dans une partie intermédiaire, disposée entre ces deux extrémités et l'ensemble des valeurs mesurées est mémorisé pour transfert aux moyens de fabrications des enveloppes.

Les données ainsi obtenues sont soit collectées sur un support de mémorisation, soit transmises directement à une machine à tricoter, pour obtenir une enveloppe personnalisée, adaptée à la morphologie du corps ou du membre et exerçant sur la longueur à traiter les contraintes de compression définies pour obtenir l'effet correcteur visualisé à l'écran, dans la phase de mesure.

Avec ce procédé, le fabricant de l'enveloppe, utilisant aujourd'hui un métier à tricoter à commande numérique, n'a plus qu'à régler le métier de manière que, lors de la fabrication de l'enveloppe, les fils élastiques soient soumis à la tension T1 relevée par la mesure dans chacune des sections de l'enveloppe qui correspondent aux sections transversales analysées sur le membre.

On notera que les valeurs mesurées prennent en compte la résistance à la pénétration procurée par la masse absorbante et sont donc obtenues avec une marge d'erreur beaucoup plus faible que celle découlant des plages de tolérances acceptées avec le système de Classes utilisées pour la fabrication des bas de contention. Il est ainsi possible d'obtenir des enveloppes fournissant des valeurs précises de contention ou compression, proche de la valeur idéale pour le traitement envisagé, et pouvant être utilisées dans de nouvelles applications exigeant une plus grande précision, par exemple avec des textiles communicants, extensibles ou non, équipés de nanocapteurs intégrés dans les fils textiles.

L'invention concerne également le dispositif pour la mise en oeuvre de ce procédé à partir d'un équipement d'imagerie médicale défini ci-dessus.

Dans le dispositif selon l'invention, les moyens de gestion et de commande comprennent aussi une commande de déclenchement de l'opération de mesure de la valeur de la pression P0 ou P1 et, dans une mémoire, un logiciel apte à exécuter l'équation E selon laquelle $T = P \times L / 2\pi$ calculant, à partir de la valeur P0 ou P1 de la pression P fournie par les moyens de mesure et de la valeur mesurée de L, la valeur T0 ou T1 de la tension devant être communiquée a des fils élastiques pour amener l'élément examiné dans le même état que celui observé à l'écran.

Dans une forme d'exécution de ce dispositif, le brassard est constitué par une bande inextensible enroulable autour du membre ou partie de corps et comporte, d'une part et près de son extrémité libre, un capteur relié électriquement à une unité de lecture et de mémorisation, faisant partie des moyens de gestion et de commande et, d'autre part et sur sa partie venant sous l'extrémité précitée quand le brassard est enroulé sur le membre ou corps, une graduation, optique ou, électronique, lisible par le capteur et indiquant la valeur de la longueur circonférentielle L du tronçon de membre sous cette graduation.

Ainsi, en fin de mesure on dispose, pour chaque section examinée du membre ou corps, dun ensemble de données comprenant des valeurs de tension en rapport avec les longueurs périphériques des sections, et permettant de se libérer des tailles et standards morphologiques. Ces données peuvent être mémorisées et utilisées plus tard ou envoyées directement à une machine à tricoter qui réalise alors une enveloppe personnalisée, tubulaire ou plane.

Cet aménagement simplifie la mesure et permet d'augmenter son automatisation, en particulier lors de la mesure des paramètres d'un enveloppe devant fournir des valeurs de contention ou de compression différentes dans diverses sections transversales du membre.

Un exemple concerne aussi une enveloppe de contention élastique tricotée à plat selon les valeurs découlant des mesures de pression dans le coussin gonflable, et conformée en manchon par accrochage de deux de ses extrémités.

L'enveloppe selon l'exemple comprend, sur sa face venant à l'extérieur du membre ou partie du corps et dans chacune de ses zones extrêmes d'accrochage, des marques ou moyens dont la mise en coïncidence, bout à bout ou par superposition, donne au manchon la même longueur circonférentielle L que celle relevée pendant la mesure des valeurs de P1 et de T1 sur le membre ou partie du corps.

Cet aménagement est indispensable, car si l'enveloppe ne présente pas sur le corps la même longueur circonférentielle que celle du le brassard au moment de la mesure, même avec les bonnes valeurs de P1 ou T1, la contention ou compression exercée ne permet pas d'amener l'élément compris dans la masse absorbante de l'interface au même état que celui observé pendant la mesure.

Dans une forme d'exécution, au moins l'un des fils élastiques assurant la tension dans l'enveloppe est muni de marques colorées qui, contrastant avec la couleur générale de cette enveloppe, sont occultées par les autres fils lors de la fabrication de l'enveloppe et n'apparaissent que quand la tension élastique passe au dessous de la valeur de seuil T0 et n'est plus apte à assurer la contention.

L'invention sera mieux comprise à l'aide de la description qui suit en référence au dessin schématique annexé dans lequel

Les figures 1 à 4 sont des copies d'écran lors de l'examen de la section transversale d'une jambe, au niveau du creux poplité, par un échographe dont la sonde est passée sur la fenêtre acoustique d'un brassard gonflable, gonflé avec une pression ayant différentes valeurs ;
Figure 5 est un schéma d'une forme d'exécution du dispositif lorsqu'il est mis en oeuvre avec un échographe ;
Figure 6 est une vue ne perspective d'une forme d'exécution d'une enveloppe de traitement, selon l'invention.

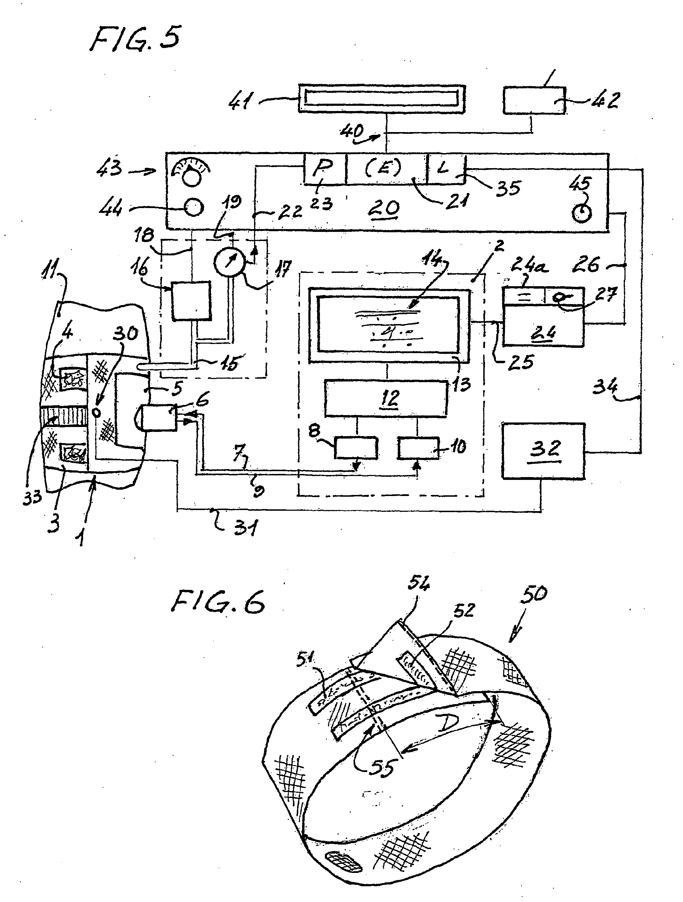

Le dispositif représenté à la figure 5 comprend, de manière générale, le brassard 1 d'un tensiomètre et un appareil échographique 2.

Le brassard 1 est composé d'une nappe 3 réalisée dans un matériau inextensible dans les conditions normales d'utilisation du brassard. Cette nappe 3, dont la longueur et la largeur sont définies selon le membre ou partie de corps à examiner, est munie de moyens d'accrochage 4 des ses extrémités, par exemple des bandes de rubans à crochets coopérant avec des bandes de rubans à bouclettes, des bandes opposées à liaison par collage ou des aimants ou bandes aimantées opposées. Comme dans un sphygmanomètre la nappe 3 est solidaire d'un coussin qui, non visible au dessin, est gonflable par un fluide, tel que de l'air, pour exercer une pression sur au moins une partie du membre examiné 11. Le brassard comporte localement une fenêtre acoustique 5 laissant passer les ondes ultrasonores émises par la sonde 6 de l'échographe.

La sonde 6 est reliée par un circuit 7 à un générateur d'ultrasons 8 et par un circuit 9 à un récepteur 10 du spectre sonore réfléchi et capté par cette sonde. Le générateur 8 et le récepteur 10 sont reliés à une unité de traitement 12 qui compare les signaux émis avec les signaux reçus et délivre, sur un écran de lecture 13, une image14 d'une zone du membre observé. De préférence, l'unité de traitement 12 fournit des images en couleur qui, selon les besoins de l'analyse, sont en deux ou trois dimensions.

Le brassard 3 est associé, par un conduit 15, à des moyens de gonflage et de dégonflage qui peuvent être constitués, soit par la traditionnelle poire avec robinet de vidange, soit, comme représenté figure 5, par un ensemble pompe et distributeur 16 avec appareil 17 de mesure de la pression dans le conduit 15, entre distributeur et coussin.

Le fonctionnement des moyens de gonflage 16 et de l'appareil de mesure 17 est commandé, par l'intermédiaire de circuits électriques respectifs 18 et 19, par une unité de gestion et de commande 20. L'information de la valeur de la pression est transmise par un circuit 22 à une mémoire 23 de l'unité 20.

A l'échographe 2 est associé un analyseur d'écran 24, relié à l'écran 13 par un circuit 25 et à l'unité de gestion 20 par un circuit 26. Cet appareil est équipé de moyens 24a de mémorisation d'au moins une image particulière présentée à l'écran et de moyens 27 de réglage de sa sensibilité. Il est apte à comparer l'image présentée à l'écran avec l'une de celles mémorisées et, dès qu'une modification de l'état de l'image à l'écran devient significative par rapport au réglage de sensibilité, à déclencher une nouvelle phase, dans un processus de mesure commandé par l'unité de gestion 20.

L'unité de gestion et de commande 20 communique par un circuit 40 avec un afficheur 41 et/ou une imprimante 42.

A ces moyens décrits dans l'état de la technique, le dispositif selon l'invention ajoute :

- des moyens de mesure de la longueur circonférentielle L de la partie du corps ou du tronçon de membre en cours d'observation et de mesure et,

- dans l'unité de gestion 20, un logiciel 21 de calcul de l'équation (E), telle que T = P x L / 2 π, donnant la valeur de la tension T, devant être communiquée aux brins élastiques de l'enveloppe de contention pour une valeur Pn de la pression de contention exercée par le coussin gonflable sur la zone observée et pour la valeur L de la longueur circonférentielle de la dite zone.

Dans la forme d'exécution représentée à la figure 5 les moyens de mesure de la circonférence de la zone observée du membre comprennent, d'une part et près de l'extrémité libre extérieure de la nappe 3 du brassard, un capteur 30 relié par un circuit électrique 31 à une unité de lecture et de mémorisation 32 et, d'autre part et sur sa partie venant sous l'extrémité précitée quand le brassard est enroulé sur le membre, une graduation 33, optique ou électronique, lisible par le capteur 30 et indiquant la valeur de la longueur circonférentielle du tronçon de membre sous cette graduation: L'unité 32 est reliée par un circuit 34 à une mémoire 35 de l'unité de gestion 20.

Enfin, l'unité de gestion et de commande 20 comprend un sélecteur 43, permettant de choisir un cycle de mesure parmi plusieurs cycles proposés, un bouton 44 de déclenchement du cycle de mesure sélectionné et un bouton 45 de déclenchement de la mesure de la pression P.

La détermination de la valeur de la pression P permettant d'obtenir l'effet attendu sur l'élément du corps examiné, tel que compression de la structure graisseuse, serrage d'un tendon ou réduction du calibre d'un vaisseau, s'effectue en plusieurs phases.

Pendant la première phase a), qui est dénommée « préparatoire », un brassard 1, de longueur et largeur adaptées aux dimensions du membre ou partie du corps devant être examiné, est mis en place sur un tronçon du membre 11. L'extrémité libre extérieure de la nappe 3 du brassard est accrochée sur l'autre extrémité de la nappe, par exemple par coopération de ses bandes de velours à crochets avec les bandes de velours à bouclettes sous jacentes. Le capteur 30 vient ainsi sur la graduation 33, mais reste sans effet.

Après sélection du cycle de mesure sur le sélecteur 43, et par exemple pour une mesure unitaire, le bouton de commande de cycle 44 est actionné. L'échographe 2 est mis en fonctionnement et l'opérateur positionne la sonde 6 de cet échographe sur la fenêtre 5 du brassard, approximativement sur la zone du corps comportant l'élément structurel à observer. Quand l'image 14 visualisée à l'écran 13 est nette, et par exemple correspond à celle de la figure 1, la phase b) de repérage à l'écran peut être engagée. Pendant celle ci l'opérateur repère sur cet écran 13 l'élément dont il doit modifier l'état, par exemple muscle profond Mp ou tendon, tout en observant l'état des autres éléments tels que veine V1 ou V2, muscle superficiel ou profond Ms, artère A1 ou A2, tendon ou tissus sous cutané T, et nerfs N1, N2.

Cette image qui représente l'état de l'élément observé pour une valeur de pression égale à la pression atmosphérique, est mémorisée cérébralement par l'opérateur et numériquement, par exemple dans la mémoire 24a de l'analyseur d'image 24.

A ce stade, la phase suivante est lancée et commence par le gonflage du brassard 1 par la pompe 16, dont le déclenchement est assuré, soit par l'opérateur, soit par l'unité de commande 20, pendant que l'opérateur continue d'appliquer la sonde sur la zone observée, tout en surveillant l'écran 13 de l'échographe.

Cette phase préparatoire est suivie par la phase de mesure d) dès que l'opérateur ou l'analyseur d'image 24 perçoit à l'écran une modification de l'état de l'élément observé, et par exemple un début de contraction d'un muscle, un début de serrage d'un tendon, un début de fermeture d'un vaisseau. Dans cette phase, l'appareil 17 mesure la valeur P0 de la pression dans le coussin gonflable du brassard 1 et, en conséquence la pression qu'il faut observer sur le tronçon de membre 11 pour passer la barrière amortissante de la masse corporelle comprenant la peau W, les tissus sous cutanés G, l'aponévrose, les muscles Ms et Mp, et les graisses.

Simultanément, l'unité 32 déclenche la mesure de la circonférence L de la zone comprimée. L'information sur la valeur de L, lue par le capteur 30 du brassard 1, est envoyée par cette unité 32 à la mémoire 35 de l'unité de gestion, tandis que l'information sur la valeur P0 de la pression P est envoyée par l'appareil de mesure 17 à la mémoire 23 de la même unité 20. Dans cette dernière, les deux valeurs de P0 et de L sont transmises au logiciel de calcul 23 qui, par l'équation E, donne la valeur de seuil de la tension T0 qu'il faut donner aux fils ou brins élastiques dans la zone de l'enveloppe de contention ou de compression destinée à recouvrir la zone analysée pour obtenir, sur le tronçon de membre, un début de pression de contention, correspondant à celle exercée par le brassard. Cette valeur est transmise à l'afficheur 41, à l'imprimante 42 et mémorisée dans l'unité 20.

Cette procédure qui permet de déterminer la valeur minimale T0 devant être donnée à la tension des fils d'une enveloppe de contention personnalisée et pour un membre déterminé, est répétée une fois sur la même zone pour obtenir la valeur T1 de la tension devant être donnée au fils, pour amener l'élément observé dans l'état désiré, et par exemple celle qu'il convient d'exercer pour réduire un muscle, serrer un tendon ou amener le vaisseau observé à une dimension, réduite par un taux allant de 10 à 100%, et par exemple de 30 %, en fonction des besoins du traitement de la circulation sanguine dans cette zone.

La mesure des valeurs P0 et P1 de la pression dans le brassard et la détermination de la valeur de la tension T0 et T1 des fils, sont répétées sur la longueur du membre par déplacement du brassard sur ce membre et répétition de la procédure ci-dessus, pour établir, après mesure aux extrémités du membre et grâce à la mémorisation des résultats obtenus, un tableau précis des tensions Tn des fils élastiques d'une enveloppe de contention ou compression, ce tableau définissant une cartographie des tensions Tn pouvant être transmise directement à l'unité de commande d'un métier à tricoter.

Il ressort de ce qui précède que le procédé et le dispositif selon l'invention permettent, par une adaptation des procédés et dispositifs connus pour la mesure de la pression sanguine, d'obtenir pour chaque individu, d'abord la valeur précise de la résistance à la compression de la zone examinée pour obtenir un changement d'état ou un effet particulier et, ensuite et pour l'application visant à réaliser une enveloppe de contention élastique, la valeur de la tension qu'il faut donner aux fils ou brins élastiques pour obtenir la contention espérée sur la zone examinée.

Dans les exemples qui précèdent, les valeurs de P0, P1 et L, sont immédiatement transformées par l'équation E, du logiciel disposé dans la mémoire 23, en valeurs de T0 et T1 pour pouvoir être transférées à la mémoire d'un métier à tricoter, mais ces valeurs P0, P1 et L peuvent être transférées directement à l'unité de traitement du métier à tricoter, comportant alors le logiciel résolvant l'équation E. Le transfert peut être effectué par internet ou par tous supports magnétiques enregistrés.

Enfin, si l'enveloppe de contention ou compression réalisée à partir des mesures relevées suivant le procédé et avec le dispositif, n'est pas en tissu élastique mais en matériau inextensible dans les conditions d'utilisation, les valeurs de référence transférées sont les valeurs des pressions P0 et P1 et la valeur L. Dans ce cas, la pression de contention ou compression de l'enveloppe est fournie par un système gonflable similaire à celui du brassard et dans lequel est introduit de l'air sous une pression égale à P.

La figure 6 montre une enveloppe compressive 50 qui est obtenue à plat par tricotage sur un machine à tricoter à commande numérique et à partir des données relevées par le procédé et le dispositif de mesure, à savoir longueur circonférentielle L de la zone examinée et à traiter et valeurs de la tension T0 et T1 dans chacune des sections mesurer.

En complément des bandes d'accrochage à crochets 51 disposées sur le dos de l'enveloppe et des bandes d'accrochage à bouclettes 52, disposées sous l'une des extrémités de la même enveloppe, celle-ci comporte des moyens matérialisant son élongation maximale pour obtenir sur le membre la compression définie.

Dans la forme d'exécution exemplaire, ces moyens sont constitués par des marques transversales 54 et 55 formées par tricotage, par exemple par des fils de couleurs, celle 54 à l'extrémité de la nappe constituant l'enveloppe 50 et celle 55 à une distance D de la première, sur la face de l'enveloppe qui est extérieure quand celle-ci est posée sur le corps.

Dans des variantes de réalisation, les marques peuvent être rapportées sur la nappe par impression ou fixation de pièces extérieures. L'enveloppe peut aussi comporter à chaque extrémité des moyens d'accrochage bout à bout et avoir une longueur adaptée pour que, après accrochage, sa longueur circonférentielle ait la valeur de L.

Grâce à cela, si lors de la pose de l'enveloppe compressive les marques sont en coïncidence, la pression exercée par l'enveloppe sur la partie de corps correspond, aux seules tolérances de fabrication près, aux valeurs mesurées dans la phase d'analyse et de mesure.

Dans une forme de réalisation exemplaire, l'un de fils assurant la tension dans l'enveloppe est muni de marques colorées qui, contrastant avec la couleur générale de cette enveloppe, sont occultées par les autres fils lors de la fabrication de l'enveloppe et n'apparaissent que quand la tension passe en dessous de la valeur de seuil T0 et n'est donc plus apte à assurer la contention.

Ainsi l'enveloppe indique quand elle ne peut plus fournir la tension justifiant son utilisation et quand elle doit être remplacée.

Bien évidemment, le procédé et le dispositif s'appliquent à la mesure de la résistance à la compression des membres ou d'une partie du corps des animaux.

## Revendications

1. Procédé de détermination des plages de pression devant être exercées par une enveloppe de contention ou compression pour parvenir aux mêmes états d'un élément du corps d'un être vivant que ceux observés et définis pendant une mesure avec un équipement d'imagerie médicale, équipement comprenant des moyens (5) d'émission de signaux, d'ondes, ou de rayonnements, un brassard (1) tel que sphygmanomètre avec fenêtre perméable aux signaux, ondes ou rayonnements, des moyens (12) de traitement des signaux, ondes ou rayonnements, captés ou réfléchis, et un écran (13) de visualisation en image des signaux traités, procédé comprenant les phases suivantes :

   - a) mise en place du brassard (1) sur la partie (11) du corps à examiner et émission des signaux, ondes ou rayonnements à travers lui,
   - b) repérage sur l'écran (13) de l'élément à analyser,
   - c) gonflage du brassard jusqu'à ce que le repérage à l'écran de l'élément en cours d'examen fasse apparaître une modification de l'état de cet élément,

- et d) lecture et mémorisation de la pression relevée dans le brassard pour parvenir à cet état,

**caractérisé en ce que** ses phases sont aménagées de la façon suivante :

- pendant la phase b), repérage sur l'écran des éléments structurels correspondant à la masse absorbante formant interface entre la peau et l'élément à analyser et pouvant comprendre les tissus sous cutanés, les tissus graisseux, les aponévroses, les fascias, les muscles, les tendons, les structures nerveuses, vasculaires et/ou ostéo-cartilagineuses,
- pendant la phase c) arrêt du gonflage dès le début de modification de l'état de l'élément analysé, puis lecture et mémorisation de la valeur de la pression PO qui traverse la masse absorbante d'interface,
- après mémorisation de la valeur P0, reprise du gonflage, jusqu'à la visualisation à l'écran de la modification souhaitée de l'état de l'élément analysé, puis arrêt de ce gonflage et lecture et mémorisation de la valeur de la pression P1 relevée dans le brassard pour parvenir à cet état,
tandis que sont ajoutées les phases suivantes
- e) mesure et mémorisation de la longueur circonférentielle L de la partie examinée du corps,
- et f) transfert des valeurs mémorisées de P0, P1 et L, soit vers un support magnétique, soit vers les moyens de fabrication d'une enveloppe élastique de contention, soit vers les moyens de mise en tension d'une enveloppe non élastique.

**2.** Procédé de détermination de plages de pression devant être exercées par une enveloppe de contention ou compression, selon la revendication 1, **caractérisé en ce que**, pour déterminer la tension qu'il faut donner aux fils élastiques d'une enveloppe élastique devant procurer un effet de contention ou de compression de valeur déterminée, il comprend, une phase supplémentaire g), réalisée pendant la mesure ou lors de la fabrication de l'enveloppe, et consistant à appliquer à l'équation E :

$$T = P \times L / 2\pi,$$

les valeurs mémorisées de P0, P1 et de L pour déterminer les valeurs de seuil T0 et optimale T1 de la tension T qu'il faut donner aux fils élastiques de l'enveloppe pour obtenir la contention ou compression produisant les mêmes effets que ceux repérés à l'écran pendant la mesure.

**3.** Procédé de détermination de plages de pression devant être exercées par une enveloppe de contention ou compression, selon l'ensemble des revendications 1 et 2, **caractérisé en ce que**, pour son application à la réalisation d'une enveloppe assurant la régulation de la circulation vasculaire, veineuse ou artérielle, il consiste, pour un même tronçon de membre (11) ou de corps :

- à mesurer d'abord la valeur P0 mini à partir de laquelle le vaisseau examiné (V

ou A) est soumis à un début de réduction de sa section,

- à déterminer par l'équation E la valeur de la tension minimale T0 à donner ultérieurement aux fils de l'enveloppe,
- à mémoriser la valeur T0 mini,
- à reprendre le gonflage du brassard (1) jusqu'à ce que la dimension transversale du vaisseau examiné, visionnée sur l'écran (13) de l'équipement d'imagerie, atteigne un taux de réduction compris entre 10 et 100 %,
- à lire et mémoriser la valeur de la pression P1 mesurée dans le brassard (1),
- et à déterminer par le calcul avec l'équation E, et avec la même valeur de L mais avec la valeur P1 de la pression, la valeur de la tension T1 qu'il faudra communiquer aux fils élastiques pour obtenir, par l'enveloppe élastique, la réduction de calibre du vaisseau perçue à l'écran.

**4.** Procédé de détermination de plages de pression devant être exercées par une enveloppe de serrage selon l'une quelconque des revendications 2 et 3 **caractérisé en ce que** les phases, respectivement, b) à d) de mesure des valeurs PO et P1 de la pression dans le brassard (1), pour un ou plusieurs états de l'élément du corps examiné, la phase e) de mesure circonférentielle L du membre ou corps et la phase g) de détermination des tensions T0 et T1 sont réalisées successivement, d'une part, sur chacune des extrémités de la partie examinée du membre (11) ou du corps et, d'autre part, au moins dans une partie intermédiaire située entre ces deux extrémités, et l'ensemble des valeurs mesurées est mémorisé pour transfert aux moyens de fabrication des enveloppes.

**5.** Dispositif pour la mise en oeuvre du procédé selon la revendication 1 et l'une quelconque des revendications 2 à 4, pour réaliser une enveloppe personnalisée de contention ou de compression, dispositif comprenant :

- des moyens (6) d'émission de signaux, d'ondes, ou de rayonnements,
- un brassard (1), apte à entourer au moins un tronçon (11) de la partie de corps à examiner, et présentant une fenêtre perméable aux signaux, ondes ou aux rayonnements,
- des moyens (12) de traitement des signaux réfléchis ou captés et envoyant sur un écran de visualisation (13) une image instantanée (14) de la partie de corps observée,
- des moyens de mémorisation (24a, 23 et 25) des images et données,
- des moyens (17) de mesure de la pression du fluide gonflant le brassard,
- et des moyens (12) de gestion et de commande des opérations, respectivement, de préparation de la mesure, de mesure et de mémorisation des images et données **caractérisé en ce que** le dispositif comprend aussi : - des moyens de mesure de la longueur circonférentielle L du tronçon de la partie de corps à examiner, et **en ce que** les moyens (12) de gestion et de commande comprennent aussi :
- une commande (45) de déclenchement de l'opération de mesure de la valeur de la pression P0 ou P1, P0 correspondant à la pression à la première modification d'état de l'élément analysé, P1 correspondant à la pression quand l'élément observé atteint l'état recherché,
- et, dans une mémoire (21), un logiciel apte à exécuter l'équation E selon laquelle $T = P \times L / 2\pi$ calculant, à partir de la valeur P0 ou P1 de la pression fournie par les moyens de mesure (17) et de la valeur mesurée de L, la valeur T0 ou T1 de la tension devant être communiquée aux fils élastiques d'une enveloppe de contention ou compression pour que celle-ci amène l'élément examiné dans le même état que celui observé à l'écran.

**6.** Dispositif selon la revendication 5 **caractérisé en ce qu'**il comporte aussi des moyens de stockage temporaire des valeurs P0, P1, L, T0 et T1 de différentes zones du brassard et des moyens permettant le transfert de ces valeurs soit vers un support magnétique, soit vers les moyens de fabrication d'une enveloppe élastique de contention, soit vers les moyens de mise en tension d'une enveloppe non élastique.

**7.** Dispositif selon la revendication 5 **caractérisé en ce que** le brassard (1) est constitué par une bande inextensible dans les conditions de mesure et comporte, d'une part et près de son extrémité libre, un capteur (30) relié électriquement à une unité de lecture et de mémorisation (32) et, d'autre part et sur sa partie venant sous l'extrémité précitée quand le brassard (1) est enroulé sur le membre, une graduation (33), optique ou, électronique, lisible par le capteur (30) et indiquant la valeur de la longueur circonférentielle L du tronçon de membre enveloppé par le brassard.

## Claims

**1.** Method for determining the ranges of pressure to be applied by a splinting or compression bandage in order to achieve the same state in an element of the body of a living being as observed and defined during measurement with medical imaging equipment, the equipment comprising means (5) of emitting signals, waves or radiation, a cuff (1) such as a sphygmanometer with a window permeable to the signals, waves or radiation, means (12) of processing the signals, waves or radiation collected or reflected and a screen (13) for viewing an image of the processed signals, the method comprising the following phases:

- a) fitting the cuff (1) over that part (11) of the body that is to be examined and emitting the signals, waves or radiation through it,
- b) identifying on the screen (13) the element that is to be analysed,
- c) inflating the cuff until the screen representation of the element in the process of being examined reveals a change in the state of this element,
- and d) reading and storing the pressure recorded in the cuff in order to achieve this state,

**characterized in that** its phases are arranged as follows:

- during phase b) structural elements corresponding to the absorbent mass that forms the interface between the skin and the element that is to be analysed and that may include subcutaneous tissue, fatty tissues, aponeuroses, fascias, muscles, tendons, nerve, vascular and/or osteo-cartilagenous structures are identified on the screen,
- during phase c) inflation is stopped as soon as the state of the analysed element starts to change, then the value of the pressure P0 passing through the absorbent interface mass is read and stored,
- after the value P0 has been stored, inflation is resumed until the desired change in the state of the element being analysed is seen on the screen, then this inflation is halted and the value of the pressure P1 recorded in the cuff in order to achieve this state is read and stored,
while the following phases are added
- e) the circumferential length L of the examined part of the body is measured and stored,
- and f) the stored values of P0, P1 and L are transferred either to a magnetic medium or to the means of manufacturing an elastic splinting bandage, or to the means of tensioning an inelastic bandage.

2. Method of determining pressure ranges to be applied by a splinting or compression bandage according to Claim 1, **characterized in that,** in order to determine the tension that has to be applied to the elastic threads of an elastic bandage that is to afford a splinting or compression effect of determined magnitude, it comprises an additional phase g), performed during measurement or during manufacture of the bandage, and that consists in applying to the equation E:

$$T = P \times L/2\pi$$

the stored values of P0, P1 and L in order to determine the threshold value T0 and the optimum value T1 for the tension T that is to be given to the elastic threads of the bandage in order to obtain splinting or compression that produces the same effects as those identified on the screen during measurement.

3. Method of determining pressure ranges to be applied by a splinting or compression bandage according to Claims 1 and 2 together, **characterized in that,** in order to apply it to the production of a bandage that regulates venous or arterial vascular circulation, it consists, for one and the same portion of limb (11) or a body:

- in first of all measuring the minimum value P0 beyond which the vessel (V or A) examined begins to see a reduction in its cross section,
- in using the equation E to determine the minimum tension value T0 to be applied later to the threads of the bandage,
- in memorizing the minimum value T0,
- in resuming inflation of the cuff (1) until the transverse dimension of the vessel being examined, as viewed on the screen (13) of the imaging equipment, reaches a level of reduction of between 10 and 100%,
- in reading and storing the value of the pressure P1 measured in the cuff (1),
- and in determining, by calculation using equation E, and using the same value of L but with the value P1 of the pressure, the value of the tension T1 that needs to be imparted to the elastic threads in order for the elastic bandage to achieve the reduction in vessel bore seen on the screen.

4. Method of determining pressure ranges to be applied by a splinting or compression bandage according to either one of Claims 2 and 3, **characterized in that** the phases b) to d) respectively of measuring the values P0 and P1 of the pressure in the cuff (1) for one or more states of the element of body examined, phase e) of taking the circumferential measurement L of the limb or body and phase g) of determining the tensions T0 and T1 are performed in succession, on the one hand, on each of the ends of the examined part of the limb (11) or of the body and, on the other hand, at least in an intermediate part situated between these two ends, and all of the measured values are stored for transfer to the bandage manufacturing means.

5. Device for implementing the method according to Claim 1 and any one of Claims 2 to 4, in order to produce a customized splinting or compression bandage, the device comprising:

- means (6) for emitting signals, waves or radiation,
- a cuff (1) capable of being fitted around at least one section (11) of the body part that is to be examined, and having a window permeable to the signals, waves or radiation,
- means (12) of processing the reflected or collected signals and sending to a display screen (13) an instantaneous image (14) of the body part observed,
- means (24a, 23 and 25) of storing the images and data,
- means (17) of measuring the pressure of the fluid with which the cuff is inflated,

- and means (12) for managing and controlling the operations of, respectively, preparing for measuring, measuring and storing the images and data,

**characterized in that** the device also comprises:

- means of measuring the circumferential length L of the portion of the body part to be examined, and **in that** the management and control means (12) also comprise:
- a command (45) to trigger the operation of measuring the value of the pressure P0 or P1, P0 corresponding to the pressure at the first change in state of the element being analysed, P1 corresponding to the pressure when the element observed has reached the desired state,
- and, in a memory (21), software capable of running the equation E whereby $T = P \times L/2\pi$ that calculates, from the value P0 or P1 of the pressure supplied by the measurement means (17) and the measured value of L, the value T0 or T1 of the tension that is to be imparted to the elastic threads of a splinting or compression bandage so that the latter brings the examined element into the same state as the state observed on the screen.

6. Device according to Claim 5, **characterized in that** it also comprises means of temporarily storing the values P0, P1, L, T0 and T1 of varying zones of the cuff and means allowing these values to be transferred either to a magnetic medium or to the means of manufacturing an elastic splinting bandage or to means of tensioning an inelastic bandage.

7. Device according to Claim 5, **characterized in that** the cuff (1) consists of a strip that cannot extend under the measurement conditions and comprises, on the one hand, near its free end, a sensor (30) electrically connected to a reading and storing unit (32) and, on the other hand, and on its part that lies under the aforementioned end when the cuff (1) is wrapped around the limb, an optical or electronic graduation (33) that can be read by the sensor (30) and that indicates the value of the circumferential length L of the portion of limb around which the cuff is wrapped.

**Patentansprüche**

1. Verfahren zum Bestimmen von Druckbereichen, bevor sie durch eine Einschnürungs- oder Kompressionshülle ausgeübt werden, um ein Element des Körpers eines lebenden Wesens in die gleichen Zustände zu bringen wie jene, die während einer Messung mit einer medizinischen Abbildungsanlage beobachtet und definiert werden, wobei die Anlage Mittel (5) zum Aussenden von Signalen, Wellen oder Strahlungen, eine Armbinde (1) wie etwa einen Blutdruckmesser mit einem für Signale, Wellen oder Strahlungen durchlässigen Fenster, Mittel (12) zur Verarbeitung der Signale, Wellen oder Strahlungen, die aufgefangen oder reflektiert werden, und einen Schirm (13) für die bildliche Anzeige der verarbeiteten Signale enthält, wobei das Verfahren die folgenden Schritte enthält:

- (a) Anordnen der Armbinde (1) an dem zu untersuchenden Abschnitt (11) des Körpers und Schicken von Signalen, Wellen oder Strahlungen durch sie hindurch,
- (b) Kennzeichnen des zu analysierenden Elements auf dem Schirm (13),
- (c) Aufblasen der Armbinde, bis die Kennzeichnung des Elements auf dem Schirm während der Untersuchung eine Modifikation des Zustands dieses Elements zeigt, und
- (d) Lesen und Speichern des erhöhten Drucks in der Armbinde, um in diesen Zustand überzugehen,

**dadurch gekennzeichnet, dass** seine Schritte folgendermaßen beschaffen sind:

- während der Phase (b) Kennzeichnen auf dem Schirm von strukturellen Elementen, die der absorbierenden Masse entsprechen, die die Grenzfläche zwischen der Haut und dem zu analysierenden Element bildet und die subkutanen Gewebe, die Fettgewebe, die Aponeurosen, die Faszien, die Muskeln, die Sehnen sowie die Nerven-, Gefäß- und/oder Knochenknorpelstrukturen enthalten können,
- während der Phase (c) Beenden des Aufblasens, sobald die Modifikation des Zustands des analysierten Elements beginnt, dann Lesen und Speichern des Wertes P0 des Drucks, der durch die absorbierende Masse der Grenzfläche wirkt,
- nach dem Speichern des Wertes P0 Wiederaufnahme des Aufblasens, bis die gewünschte Modifikation des Zustands des analysierten Elements auf dem Bildschirm angezeigt wird, dann Beenden dieses Aufblasens und Lesen und Speichern des Wertes P1 des erhöhten Drucks in der Armbinde, um in diesen Zustand überzugehen,

wobei die folgenden Schritte hinzugefügt sind:

- e) Messen und Speichern der Umfangslänge L des untersuchten Abschnitts des Körpers, und
- f) Übertragen der gespeicherten Werte P0, P1 und L entweder an einen magnetischen Träger oder an Mittel zum Herstellen einer elastischen Einschnürungshülle oder an Mittel zum Versetzen einer nicht elastischen Hülle in Spannung.

**2.** Verfahren zum Bestimmen von Druckbereichen, bevor sie durch eine Einschnürungs- oder Kompressionshülle ausgeübt werden, nach Anspruch 1, **dadurch gekennzeichnet, dass** es zum Bestimmen der Spannung, die den elastischen Fäden einer elastischen Hülle verliehen werden muss, bevor eine Einschnürungs- oder Kompressionswirkung mit dem bestimmten Wert erhalten wird, einen zusätzlichen Schritt g) enthält, der während des Messens oder bei der Herstellung der Hülle ausgeführt wird und darin besteht, in die Gleichung E:

$$T = P \times L/2\pi$$

die gespeicherten Werte P0, P1 und L einzugeben, um den Schwellenwert T0 und den optimalen Wert T1 der Spannung T zu bestimmen, die den elastischen Fäden der Hülle verliehen werden müssen, um die Einschnürung oder Kompression zu erhalten, die die gleichen Wirkungen wie jene erzeugt, die während der Messung auf dem Bildschirm markiert worden sind.

**3.** Verfahren zum Bestimmen von Druckbereichen, bevor sie durch eine Einschnürungs- oder Kompressionshülle ausgeübt werden, nach Anspruch 1 und 2, **dadurch gekennzeichnet, dass** es für seine Anwendung zur Herstellung einer Hülle, die die Regulierung des Gefäß-, Venen oder Arterienkreislaufs gewährleistet, für denselben Teil des Körperglieds (11) oder des Körpers darin besteht:

- zunächst den Wert P0 mini zu messen, anhand dessen das untersuchte Gefäß (V oder A) einem Beginn der Reduzierung seines Querschnitts unterworfen wird,
- durch die Gleichung E den Wert der minimalen Spannung T0 zu bestimmen, der den Fäden der Hülle am Ende verliehen werden soll,
- den Wert T0 mini zu speichern,
- das Aufblasen der Armbinde (1) wiederaufzunehmen, bis die transversale Abmessung des untersuchten Gefäßes, das auf dem Schirm (13) der Bilderzeugungsanlage dargestellt wird, ein Reduktionsverhältnis erreicht, das im Bereich von 10 bis 100 % liegt,
- den Wert P1 des Drucks, der in der Armbinde (1) gemessen wird, zu lesen und zu speichern, und
- durch Berechnung anhand der Gleichung E und anhand desselben Werts für L, jedoch mit dem Wert P1 des Drucks den Wert T1 der Spannung zu bestimmen, der an die elastischen Fäden übertragen werden muss, um mit der elastischen Hülle die auf dem Schirm wahrgenommene Durchmesserreduzierung des Gefäßes zu erhalten.

**4.** Verfahren zum Bestimmen von Druckbereichen, bevor sie durch eine Einschnürungs- oder Kompressionshülle ausgeübt werden, nach einem der Ansprüche 2 und 3, **dadurch gekennzeichnet, dass** die Schritte b) bis d) des Messens der Werte P0 und P1 des Drucks in der Armbinde (1) für einen oder mehrere Zustände des untersuchten Körperteils bzw. der Schritt e) des Messens des Umfangs L des Körperteils oder des Körpers bzw. der Schritt g) des Bestimmens der Spannungen T0 und T1 nacheinander ausgeführt werden, einerseits an jedem der Enden des untersuchten Abschnitts des Körperteils (11) oder des Körpers und andererseits wenigstens in einem Zwischenabschnitt, der sich zwischen den zwei Enden befindet, wobei die Gesamtheit der gemessenen Werte gespeichert wird, um an die Mittel zum Herstellen der Hüllen übertragen zu werden.

**5.** Vorrichtung für die Ausführung des Verfahrens nach Anspruch 1 und einem der Ansprüche 2 bis 4, um eine persönliche Einschnürungs- oder Kompressionshülle herzustellen, wobei die Vorrichtung enthält:

- Mittel (6) zum Aussenden von Signalen, Wellen oder Strahlungen,
- eine Armbinde (1), die wenigstens ein Teilstück (11) des zu untersuchenden Körperabschnitts umgeben kann und ein für die Signale, Wellen oder Strahlungen durchlässiges Fenster aufweist,
- Mittel (12) zum Bearbeiten der reflektierten oder eingefangenen Signale und zum Senden eines momentanen Bildes (14) des beobachteten Körperabschnitts zu einem Anzeigeschirm (13),
- Mittel (24a, 23 und 25) zum Speichern von Bildern und Daten,
- Mittel (17) zum Messen des Drucks des die Armbinde aufblasenden Fluids, und

- Mittel (12) zum Leiten und Steuern von Operationen des Vorbereitens der Messung bzw. des Messens bzw. des Speicherns der Bilder und Daten,

**dadurch gekennzeichnet, dass** die Vorrichtung außerdem enthält:

- Mittel zum Messen der Umfangslänge L des Teilstücks des zu untersuchenden Körperabschnitts,
und dass die Mittel (12) zum Leiten und Steuern außerdem enthalten:
- eine Steuerung (45) des Auslösens der Operation des Messens des Druckwertes P0 oder P1; wobei P0 dem Druck bei der ersten Modifikation des Zustands des analysierten Elements entspricht und P1 dem Druck entspricht, wenn das beobachtete Element seinen gesuchten Zustand erreicht, und
- in einem Speicher (21) Software, die die Gleichung E ausführen kann und gemäß $T = P \times L/2\pi$ ausgehend von dem Wert P0 oder P1 des von den Messmitteln (17) gelieferten Drucks und von dem gemessenen Wert L den Wert T0 oder T1 der Spannung berechnet, bevor er an die elastischen Fäden einer Einschnürungs- oder Kompressionshülle übermittelt wird, damit diese das untersuchte Element in denselben Zustand wie jenen, der auf dem Schirm beobachtet worden ist, bringt.

6. Vorrichtung nach Anspruch 5, **dadurch gekennzeichnet, dass** sie außerdem Mittel zum vorübergehenden Speichern der Werte P0, P1, L, T0 und T1 für verschiedene Zonen der Armbinde und Mittel, die die Übertragung dieser Werte entweder an einen magnetischen Träger oder an die Mittel zur Herstellung einer elastischen Einschnürungshülle oder an die Mittel zum Versetzen einer nicht elastischen Hülle in Spannung ermöglichen, enthält.

7. Vorrichtung nach Anspruch 5, **dadurch gekennzeichnet, dass** die Armbinde (1) durch ein Band gebildet ist, das unter den Messbedingungen nicht ausdehnbar ist und einerseits in der Nähe seines freien Endes einen Sensor (30) enthält, der mit einer Lese- und Speichereinheit (32) elektrisch verbunden ist, und andererseits an seinem Teil, das sich unter dem genannten Ende befindet, wenn die Armbinde (1) um das Körperteil gewickelt ist, eine optische oder elektronische Skala (33) besitzt, die durch den Sensor (30) gelesen werden kann und den Wert der Umfangslänge L des Teils des Körperteils, um den die Armbinde gewickelt ist, angibt.

FIG.1

FIG.2

FIG.3

FIG.4

FIG.5

FIG.6

**EP 1 991 125 B1**

## RÉFÉRENCES CITÉES DANS LA DESCRIPTION

**Documents brevets cités dans la description**

- WO 0103584 A

- WO 9855072 A